# EUROPEAN PATENT APPLICATION

(11) **EP 4 082 613 A1**
(43) Date of publication of application: **02.11.2022**
(21) Application number: 20907438.4
(22) Date of filing: 08.10.2020
(51) Int. Cl.: A61P 9/10, A61P 29/00, A61P 43/00, A61P 17/02, C12N 5/078, A61K 35/15, A61K 35/28, A61K 35/51

(54) **CELL GROUP AND METHOD FOR ACQUIRING SAME**

(30) Priority: 23.12.2019 JP 2019231606
(71) Applicant: Juntendo Educational Foundation, Tokyo 113-8421 (JP); Reeir, Inc., Tokyo 108-0075 (JP)
(72) Inventor: TANAKA, Rica, Tokyo 113-8421 (JP); KOGA, Minako, Washington, D.C. 20006 (US)
(74) Representative: Mewburn Ellis LLP
(86) International application number: PCT/JP2020/039003
(87) International publication number: WO 2021/131261

(57) **Abstract**

The present invention provides a cell population and a method of obtaining the same. The cell population of the present invention is obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

## Description

### TECHNICAL FIELD

The present invention relates a cell population and a method of obtaining the same. The cell population of the present invention is obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor. The present invention also relates to a therapeutic composition for ischemic diseases, inflammatory diseases, or refractory wounds (or non-healing wounds, or chronic wounds) comprising a cell population obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

### BACKGROUND ART

Recently, ischemic diseases have been treated with cell transplantation therapy using mononuclear bone marrow cells and endothelial progenitor cells (EPCs) derived from stem cells from peripheral blood. Therefore, there is a demand particularly for a technique to culture EPCs in large quantities. A method of expanding endothelial progenitor cells in vitro from CD34 and/or CD133 positive cells has made it possible to provide a technique for efficiently culturing EPCs (Patent Literature 1). Moreover, the analysis of dynamics in the differentiation of vascular endothelial cells has revealed that there are endothelial cell-like large colony (differentiated EPC colony)-forming cells and endothelial cell-like small colony (undifferentiated EPC colony)-forming cells and allows the prediction and determination of therapeutic efficacy of cell transplantation (Patent Literature 2). A method of efficiently expanding CD34 and/or CD133 positive cells from mononuclear bone marrow cells has been also provided (Patent Literature 3).

International Publication No. WO 2014/0561154 describes a method of expanding a cell population enriched for endothelial progenitor cells or immune tolerance-inducing anti-inflammatory cells from a fraction of mononuclear cells derived from bone marrow, umbilical cord blood, or peripheral blood, which may be hereinafter referred to as "QQ-MNC method". This method comprises expanding a cell population comprising EPCs in vitro by culturing mononuclear cells in a serum-free medium containing five factors: (1) stem cell factor (SCF), (2) interleukin-6 (IL-6), (3) FMS-like tyrosine kinase 3 ligand (FL), (4) thrombopoietin (TPO), and (5) vascular endothelial growth factor (VEGF).

### CITATION LIST

### PATENT LITERATURE

PTL 1: International Publication No. WO 2006/090882
PTL 2: International Publication No. WO 2006/090886
PTL 3: International Publication No. WO 2006/093172
PTL 4: International Publication No. WO 2014/0561154

### SUMMARY OF INVENTION

### TECHNICAL PROBLEM

As a result of intensive research, the present inventors have successfully obtained a novel cell population that has abilities to regenerate blood vessels and to heal wounds and is different from a cell population obtained by the QQ-MNC method, by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor, and thus have completed the present invention.

The present invention aims to provide a cell population. The cell population of the present invention is obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

The present invention also aims to provide a method of obtaining a cell population. The method of the present invention comprises culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

The present invention further aims to provide a therapeutic composition for ischemic diseases, inflammatory diseases, or refractory wounds. The composition of the present invention comprises a cell population obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

### SOLUTION TO PROBLEM

The present invention includes, but is not limited to, the following embodiments:
[Embodiment 1] A cell population obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.
[Embodiment 2] The cell population according to Embodiment 1, wherein the medium contains three or four factors selected from stem cell factor, interleukin-6, FMS-like tyrosine kinase 3 ligand, thrombopoietin, and vascular endothelial growth factor.
[Embodiment 3] The cell population according to Embodiment 1 or 2, wherein the serum is bovine or human serum.
[Embodiment 4] The cell population according to any one of Embodiments 1 to 3, wherein the serum is contained in the medium at a concentration from 0.5 vol% to 10 vol%.
[Embodiment 5] The cell population according to any one of Embodiments 1 to 4, wherein the total of CD206(+), CD34(+), or CD3(+) cells collectively represents 60% or more of the cell population, and CCR2(-) cells represent 95% or more of the cell population.
[Embodiment 6] The cell population according to Embodiment 5, wherein CXCR4(+) cells in CD206(+) cells represent 50% or more of the cell population.
[Embodiment 7] The cell population according to Embodiment 5 or 6, wherein CXCR4(+) cells in CD206(+) cells represent 80% or more of the cell population.
[Embodiment 8] A method of obtaining the cell population according to any one of Embodiments 1 to 7, comprising culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.
[Embodiment 9] The method according to Embodiment 8, wherein the total of CD206(+), CD34(+), or CD3(+) cells collectively represents 60% or more of the cell population, and CCR2(-) cells represent 95% or more of the cell population.
[Embodiment 10] The method according to Embodiment 8 or 9, wherein CXCR4(+) cells in CD206(+) cells represent 50% or more of the cell population.
[Embodiment 11] The method according to any one of Embodiments 8 to 10, wherein the medium contains three or four factors selected from stem cell factor, interleukin-6, FMS-like tyrosine kinase 3 ligand, thrombopoietin, and vascular endothelial growth factor.
[Embodiment 12] The method according to any one of Embodiments 8 to 11, wherein the serum is fetal bovine serum.
[Embodiment 13] The method according to any one of Embodiments 8 to 12, wherein the serum is contained in the medium at a concentration from 0.5 vol% to 10 vol%.
[Embodiment 14] A therapeutic composition for ischemic diseases, inflammatory diseases, or refractory wounds, comprising the cell population according to any one of Embodiments 1 to 7.
[Embodiment 15] The therapeutic composition according to Embodiment 14, wherein the ischemic disease is limb ischemia.
[Embodiment 16] The therapeutic composition according to Embodiment 14 or 15, wherein the ischemic disease is limb ischemia with ulcers.
[Embodiment 17] The therapeutic composition according to any one of Embodiments 14 to 16, promoting vascularization and/or wound healing.

### BRIEF DESCRIPTION OF DRAWINGS

Fig. 1 shows the result of fractionation of cells composing RE-01 prepared from peripheral blood from a healthy human by flow cytometry (FACS). The FACS result of peripheral blood mononuclear cells before culturing is also shown as a comparative example.
Fig. 2 shows the results of analyzing cells composing RE-01 prepared from peripheral blood from a healthy human different from the individual used in Fig. 1 by flow cytometry (FACS).
Fig. 3 shows the results of analyzing cells composing RE-01 prepared from peripheral blood from a 53-years old female diabetic patient by flow cytometry (FACS). The cell population (MNC-QQ) obtained from the culturing in a serum-free medium containing five factors was used as a comparative example.
Fig. 4 shows the results of determining the ability of RE-01 to form blood vessels, using human umbilical vein endothelial cells (HUVECs). As comparative examples, MNC-QQ was used instead of RE-01, and HUVECs were used alone. Fig. 4A shows the change of the number of formed lumens over time from the start of culturing. Fig. 4B shows the number of formed lumens after 2.75 hours from the start of culturing. Fig. 4C shows the number of fluorescently labeled test cells incorporated in the lumen structure after 2.75 hours from the start of culturing.
Fig. 5 shows the results of fractionation of cells composing RE-01 prepared from peripheral blood from a patient with severe lower limb ischemia by flow cytometry (FACS).

### DESCRIPTION OF EMBODIMENTS

### 1. Cell population

The present invention relates to a cell population.

The cell population of the present invention is obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

The cell population is obtained by culturing mononuclear cells derived from bone marrow, umbilical cord blood, or peripheral blood. The "mononuclear cells" that are used to obtain the cell population collectively refer to cells that each have a round nucleus and are included in peripheral blood, bone marrow, umbilical cord blood, or the like. The mononuclear cells include lymphocytes, monocytes, macrophages, endothelial progenitor cells, and hematopoietic stem cells. For example, mononuclear cells are obtained by harvesting bone marrow, umbilical cord blood, or peripheral blood from any animals and transferring them to, for example, a blood collection tube for separating mononuclear cells or subjecting them to density gradient centrifugation to isolate a fraction of mononuclear cells. Any density gradient centrifugation that forms a fraction of mononuclear cells may be used. For example, Histopaque-1077 (Sigma-Aldrich) may be used.

Bone marrow, umbilical cord blood, and peripheral blood may be derived from any animal species. The animal species include mammals in general, including human, to which cell transplantation therapy for diseases such as ischemic diseases, inflammatory diseases, or refractory wounds may be applied. In light of the purpose of clinical application, the animal species is preferably human.

Bone marrow, umbilical cord blood, and peripheral blood may be derived from any subjects. In an embodiment, the subjects may be, for example, a healthy individual, a diabetic patient, or a patient with severe lower limb ischemia.

Stem cell factor (SCF) is a glycoprotein that consists of 248 amino acids and has a molecular weight of about 30,000. Soluble and membrane-associated forms are produced from alternative splicing. Any types of SCF may be used as long as it is useful for culturing mononuclear cells to obtain the cell population. SCF is preferably in a soluble form. SCF may be derived from any sources. SCF is preferably, but not limited to, a recombinant which is likely to be stably available, particularly preferably a human recombinant. Some commercially available SCF products are known. The concentration of SCF in a culture medium will vary depending on SCF to be used, and any concentrations useful for culturing mononuclear cells may be used. The concentration of human recombinant SCF is, for example but not limited to, 10 to 1000 ng/mL, preferably 50 to 500 ng/mL, more preferably about 100 ng/mL.

Interleukin-6 (IL-6) is a glycoprotein that has been isolated as a factor inducing terminal differentiation of B cells into antibody-producing cells and has a molecular weight of 210000. IL-6 is generally known to be involved in immune responses, proliferation and differentiation of hematopoietic and neural cells, acute phase responses, and the like. IL-6 that is used to obtain the cell population is not particularly limited and is appropriately selected. When used for culturing human mononuclear cells, IL-6 is preferably human IL-6, particularly preferably a recombinant which is likely to be stably available. Some commercially available IL-6 products are known. The concentration of IL-6 in a culture medium will vary depending on IL-6 to be used, and any concentrations useful for culturing mononuclear cells may be used. The concentration of human recombinant IL-6 is, for example but not limited to, 1 to 500 ng/mL, preferably 5 to 100 ng/mL, more preferably about 20 ng/mL.

FMS-like tyrosine kinase 3 ligand (FL) is known to be a ligand for receptor tyrosine kinase that plays an important role in the control of early hematopoiesis. Several products are known to be produced from alternative splicing and have been reported to stimulate the proliferation of hematopoietic stem cells. Any types of FL may be used as long as it is useful for culturing mononuclear cells to obtain the cell population. Some commercially available FL products are known. The concentration of FL in a culture medium will vary depending on FL to be used, and any concentrations useful for culturing mononuclear cells may be used. The concentration of human recombinant Flt-3 ligand is, for example but not limited to, 10 to 1000 ng/mL, preferably 50 to 500 ng/mL, more preferably about 100 ng/mL.

Thrombopoietin (TPO) is a hematopoietic cytokine and is known to specifically act on a process of producing megakaryocytes from hematopoietic stem cells and to promote the production of megakaryocytes. TPO that is used to obtain the cell population may be derived from any sources. TPO is preferably a recombinant which is likely to be stably available, particularly preferably a human recombinant. Some commercially available TPO products are known. The concentration of TPO in a culture medium will vary depending on TPO to be used, and any concentrations useful for culturing mononuclear cells may be used. The concentration of human recombinant TPO is, for example but not limited to, 1 to 500 ng/mL, preferably 5 to 100 ng/mL, more preferably about 20 ng/mL.

Vascular endothelial growth factor (VEGF) is a growth factor that specifically acts on endothelial progenitor cells (EPCs) and is known to be mainly produced in perivascular cells. Several VEGF proteins having different sizes are produced from alternative splicing. Any types of VEGF may be used as long as it allows EPC colony formation to obtain the cell population. VEGF is preferably VEGF165. VEGF may be derived from any sources. VEGF is preferably a recombinant which is likely to be stably available, particularly preferably a human recombinant. Some commercially available VEGF products are known. The concentration of VEGF in a culture medium will vary depending on VEGF to be used, and any concentrations useful for culturing mononuclear cells may be used. The concentration of human recombinant VEGF165 is, for example but not limited to, about 5 to 500 ng/mL, preferably about 20 to 100 ng/mL, more preferably about 50 ng/mL.

Various factors that are added to a culture medium used to culture mononuclear cells may be derived from, but not limited to, the same animal from which the mononuclear cells are derived. When the source of mononuclear cells is the same as that of various factors, a suitable cell culture for allotransplantation such as allogeneic transplantation will be provided. The use of mononuclear cells derived from an individual scheduled to receive cell transplantation will also provide a suitable cell culture for syngeneic transplantation.

A culture medium for culturing mononuclear cells can be prepared by dissolving each component as described above in the culture medium at a predetermined concentration or diluting previously prepared concentrates (stock solutions) for each component in a culture medium at a predetermined concentration. For example, a culture medium can be prepared by dissolving appropriate components in a commercially available culture medium at a predetermined concentration and then subjecting it to sterilization such as filter sterilization, or aseptically adding or diluting stock solutions sterilized by filter sterilization or other sterilization in a commercially available culture medium. Filter sterilization may be performed according to any method commonly conducted in the art and is performed, for example, using 0.22 µm or 0.45 µm Millipore filter.

The "culture medium" used in the present invention may be any medium that is commonly used in the art and for example, is known to be a growth medium for hematopoietic stem cells. Examples of the minimal essential medium used as a culture medium include Stemline II, DMEM, MEM, IMDM, RPMI, SCGM, and EBM.

The medium for culturing mononuclear cells to obtain the cell population contains serum in addition to four or less of factors selected from the group consisting of stem cell factor, interleukin-6, FMS-like tyrosine kinase 3 ligand, thrombopoietin, and vascular endothelial growth factor.

Any type of serum may be used. In an embodiment, the serum is, but not limited to, bovine or human serum. In an embodiment, the serum is fetal bovine serum and/or human serum albumin. Any concentrations of the serum may be used in the medium. In an embodiment, the serum is contained in the medium at 0.1 vol% or more, 0.3 vol% or more, or 0.5 vol% or more. The serum concentration in the medium has no upper limit unless otherwise specified. In an embodiment, the serum is contained at 30 vol% or less, 20 vol% or less, 10 vol% or less, or 5 vol% or less. The serum is contained in the medium at a concentration, but not limited to, from 0.1 vol% to 20 vol% or from 0.5 vol% to 10 vol%.

The culturing of mononuclear cells is performed by adding a cell suspension comprising mononuclear cells to a medium containing the factors and serum as described above. The cell suspension may be a body fluid comprising mononuclear cells (e.g., bone marrow fluid, umbilical cord blood, and peripheral blood). Mononuclear cells may be cultured under any conditions and can be cultured under conditions commonly used in the art. Mononuclear cells are cultured, for example but not limited to, in an atmosphere of 5% CO2 at about 37°C. Non-limiting examples of the culture period include 3 days or more, and 5 days or less, 6 days or less, 7 days or less, or 10 days or less. For example, the culture period is from 3 to 10 days or from 3 to 6 days. Any concentrations of mononuclear cells that allow the culturing of mononuclear cells may be used in a medium. For example, the concentration is about 0.1 to 10 × 10⁶ cells/ml, more preferably about 0.5 to 5 × 10⁶ cells/ml.

As used herein, the "cell population" collectively refers to cells obtained by culturing mononuclear cells in a medium containing the factors and serum as described above.

A culture medium that is used to culture mononuclear cells contains four or less factors selected from the group consisting of stem cell factor, interleukin-6, FMS-like tyrosine kinase 3 ligand, thrombopoietin, and vascular endothelial growth factor.

In an embodiment, the medium contains two or more or three or more factors selected from stem cell factor, interleukin-6, FMS-like tyrosine kinase 3 ligand, thrombopoietin, and vascular endothelial growth factor. In an embodiment, the medium contains two to four factors selected from the five factors. In an embodiment, the medium contains three or four factors selected from stem cell factor, interleukin-6, FMS-like tyrosine kinase 3 ligand, thrombopoietin, and vascular endothelial growth factor.

A culture medium that is used to culture mononuclear cells and thus a culture medium that is used in the culture method of the present invention contain, for example but not limited to, i) a combination of FMS-like tyrosine kinase 3 ligand, thrombopoietin, and vascular endothelial growth factor or ii) a combination of stem cell factor, FMS-like tyrosine kinase 3 ligand, and vascular endothelial growth factor. More preferably, the medium contains a combination of about 80 to 120 ng/ml FMS-like tyrosine kinase 3 ligand, about 15 to 25 ng/ml thrombopoietin, and about 40 to 60 ng/ml vascular endothelial growth factor.

In an embodiment, a cell population obtained by culturing mononuclear cells derived from bone marrow, umbilical cord blood, or peripheral blood in a medium containing serum and four or less factors selected from the group consisting of stem cell factor, interleukin-6, FMS-like tyrosine kinase 3 ligand, thrombopoietin, and vascular endothelial growth factor comprises CD206(+), CD34(+), or CD3(+) cells more than a pre-culture cell population of mononuclear cells. The culturing may increase the proportion of CD206(+), CD34(+), or CD3(+) cells, but not limited to, 1.5-times or more, 2-times or more, or 3-times or more.

The total of CD206(+), CD34(+), or CD3(+) cells in the cell population collectively represents, but not limited to, 50% or more, 60% or more, 70% or more, 80% or more, or 90% or more of the entire cell population. The cell population also comprises CCR2(-) cells more than a pre-culture cell population of mononuclear cells. The culturing may increase the proportion of CCR2(-) cells, but not limited to, 1.5-times or more, 2-times or more, or 3-times or more. CCR2(-) cells in the cell population represent, but not limited to, 60% or more, 80% or more, 90% or more, 95% or more of the entire cell population. By way of non-limiting example, in an embodiment, CD206(+), CD34(+), and CD3(+) cells represent 60% or more, and CCR2(-) cells represent 95% or more.

When mononuclear cells derived from bone marrow, umbilical cord blood, or peripheral blood are cultured in a medium containing serum and four or less of factors selected from the group consisting of stem cell factor, interleukin-6, FMS-like tyrosine kinase 3 ligand, thrombopoietin, and vascular endothelial growth factor, a cell population comprising more CD206(+), CD34(+), and CD3(+) cells can be stably produced. Moreover, the produced cell population has a markedly higher proportion of CXCR4 positive cells in CD206 positive cells including anti-inflammatory macrophage M2 than a cell population obtained by the QQ-MNC method. The present inventors found that the culturing provides, but not limited to, the following efficacy:
An increase in CD206 positive cells including anti-inflammatory macrophage M2;
An increase in CD34 positive cells including endothelial progenitor cells (EPCs) and CD133 positive cells; and
An increase in T cells, particularly CD3 positive cells such as helper T cells and angiogenic T cells.

In contrast, it was found that CCR2 positive cells which are inflammatory monocytes/macrophages, B cells (CD19 positive cells), and NK cells (CD56 positive cells) are decreased.

In an embodiment, a cell population obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor has, but not limited to, a higher recovery than a cell population obtained by the QQ-MNC method. Moreover, the cell population obtained from such culturing has a high survival rate and has high stability after recovery. Such cell population will provide a higher therapeutic efficacy.

In an embodiment, a cell population obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor comprises CXCR4(+) cells in CD206(+) cells more than a pre-culture cell population of mononuclear cells. The culturing may increase the proportion of CXCR4(+) cells in CD206(+) cells, but not limited to, 1.5-times or more, 2-times or more, or 3-times or more. CXCR4(+) cells in CD206(+) cells represent, but not limited to, 50% or more, 60% or more, 70% or more, or 80% or more. In an embodiment, CXCR4(+) cells in CD206(+) cells represent 50% or more. In an embodiment, CXCR4(+) cells in CD206(+) cells represent 80% or more.

The cell population of the present invention comprises particularly CXCR4(+) cells in CD206(+) cells more than a cell population obtained by the QQ-MNC method.

### 2. Method of obtaining a cell population

The present invention also relates to a method of obtaining a cell population.

The method of the present invention comprises culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

The "cell population", "mononuclear cells", each factor of the "stem cell factor, interleukin-6, FMS-like tyrosine kinase 3 ligand, thrombopoietin, and vascular endothelial growth factor", "serum", "medium", and the like are as described in the section "1. Cell population".

A proportion of CD206(+), CD34(+), and CD3(+) cells in a cell population, a proportion of CCR2(-) cells, and a proportion of CXCR4(+) cells in CD206(+) cells are also as described in the section "1. Cell population".

In an embodiment, CD206(+), CD34(+), and CD3(+) cells represent 80% or more of a cell population obtained by the method, and CCR2(-) cells represent 95% or more.

In an embodiment, CXCR4(+) cells in CD206(+) cells represent 50% or more of a cell population obtained by the method.

In an embodiment of the method, the medium contains three or four factors selected from stem cell factor, interleukin-6, FMS-like tyrosine kinase 3 ligand, thrombopoietin, and vascular endothelial growth factor.

In an embodiment of the method, the serum is bovine or human serum. In an embodiment, the serum is fetal bovine serum. In an embodiment, the serum is contained in a medium at a concentration from 0.5 vol% to 10 vol%.

### 3. Therapeutic composition

The present invention further relates to a therapeutic composition for ischemic diseases, inflammatory diseases, or refractory wounds. The therapeutic composition of the present invention comprises a cell population as described in the section "1. Cell population". Mononuclear cells from which the cell population is derived may be derived from a subject that will receive the therapeutic composition (autologous) or a subject that will not receive the therapeutic composition (allogeneic).

"Ischemic disease" is a disease caused by histological damage, such as cell degeneration, atrophy, and fibrogenesis, that results from a decrease in blood volume leading to a decrease in blood flow in tissues. Ischemia is largely divided into occlusive ischemia, compressive ischemia, spastic ischemia, and compensated ischemia on the basis of its causes. Prolonged ischemia will cause cell degeneration, atrophy, and fibrogenesis. "Ischemic disease" includes limb ischemia, ischemic ulcer, ischemic heart disease, and cerebral infarction. "Cerebral infarction" (or encephalomalacia) is classified into cerebral thrombosis and cerebral embolism which refer to necrosis or necrosis-like condition in the brain tissue caused by cerebral ischemia and thus an insufficient supply of oxygen or nutrients due to obstruction or stenosis of arteries supplying blood and oxygen to the brain. "Limb ischemia" is a disease in which an artery supplying blood to limbs is stenosed or occluded. Exacerbation of arteriosclerosis obliterans will lead to severe limb ischemia, cause symptoms such as pain and refractory ulcers, and at worst, may require amputation. In an embodiment, limbs represent, but are not limited to, lower limbs.

In an embodiment, ischemic disease is, but not limited to, limb ischemia. Ischemic disease is limb ischemia with ulcers.

"Inflammatory diseases" collectively refer to diseases that develop defects, such as tissue injuries, from any causes. Inflammatory diseases include Crohn's disease, liver cirrhosis, hepatitis, ulcerative colitis, and inflammatory bowel disease.

The therapeutic composition promotes vascularization and/or wound healing. Therefore, the therapeutic composition can be applied to diseases that can be treated with vascularization and/or wound healing. Examples of the "diseases that can be treated with vascularization and/or wound healing" include ischemic diseases (e.g., ischemic heart diseases such as myocardial infarction and angina pectoris, lower limb ischemia such as lower limb ischemic arteriosclerosis, and Buerger disease) and vascular injury. The therapeutic composition can be also used to heal wounds such as skin ulcers or to create artificial blood vessels. The efficacy of the therapeutic composition applied can be confirmed by any known methods. For example, when the disease that can be treated with vascularization is lower limb ischemic disease, the post-transplant therapeutic efficacy can be assessed by determining, for example, blood flow in lower limbs and necrosis improvement rate. An increase in blood flow can be determined by measuring the value of laser Doppler imaging analysis. The necrosis improvement rate can be determined as a limb salvage score by visual observation.

The therapeutic composition may be used in any form. The therapeutic composition may be a cell population itself that is an active ingredient or a suspension of a cell population in a liquid medium. The liquid medium may be any liquid that can be infused to human. Examples of the liquid medium include isotonic electrolyte infusion, phosphate buffer, physiological saline, and serum-free medium, DMEM. The liquid medium may also contain compounds desirable for survival of cells, such as albumin. Preferably, a compound containing albumin is serum derived from a patient. When cryopreserved, the therapeutic composition may be a suspension in a cryoprotectant solution or other solutions.

The therapeutic composition may be applied to any subjects that require the treatment of ischemic diseases, inflammatory diseases, or refractory wounds. Non-limiting examples of the subjects include human, monkey, chimpanzee, dog, cat, cattle, horse, mouse, and guinea pig.

### 4. Kits

The present invention further relates to a kit for obtaining a cell population by culturing mononuclear cells derived from bone marrow, umbilical cord blood, or peripheral blood.

The kit comprises a medium containing serum and four or less of factors selected from the group consisting of stem cell factor, interleukin-6, FMS-like tyrosine kinase 3 ligand, thrombopoietin, and vascular endothelial growth factor. Each of the factors and serum may be added into a medium in advance or may be stored in separate containers and added into a medium in use.

The "cell population", "mononuclear cells", each factor of the "stem cell factor, interleukin-6, FMS-like tyrosine kinase 3 ligand, thrombopoietin, and vascular endothelial growth factor", "serum", "medium", and the like are as described in the section "1. Cell population".

### 5. Treatment methods and others

The present invention further relates to a method of treating ischemic diseases, inflammatory diseases, or refractory wounds, comprising applying a cell population as described in the section "1. Cell population" to a subject in need thereof.

The present invention also relates to use of a cell population as described in the section "1. Cell population" in a method of treating ischemic diseases, inflammatory diseases, or refractory wounds, comprising applying the cell population to a subject in need thereof, or use of the cell population in a therapeutic composition for ischemic diseases, inflammatory diseases, or refractory wounds.

The "ischemic diseases", "inflammatory diseases", or "refractory wounds", "embodiments of applying the cell population", "applied subjects", and the like are as described in the section "3. Therapeutic composition".

### EXAMPLES

The present invention will be described in detail below in reference to the Examples but is not limited to the Examples. Those skilled in the art could readily modify and alter the present invention on the basis of the description in the specification, and the modification and alteration are within the technical scope of the present invention.

### Example 1: Culturing of RE-01 cell population

In this Example, mononuclear cells were isolated from peripheral blood and cultured.

### (1) Isolation of mononuclear cells

One hundred mL of peripheral blood was collected from volunteers of healthy humans or diabetic patients into a BD Vacutainer(R) CPT(TM) (Becton, Dickinson and Company) blood collection tube for separating mononuclear cells. After blood collection, the blood collection tube was centrifuged as such, transported, received, and then stored under refrigeration until the start of culturing. The upper layer, which contained mononuclear cells and blood plasma, over the gel barrier in the CPT(TM) blood collection tube was collected into a centrifuge tube. The inside of the blood collection tube over the gel barrier was rinsed with a small volume of EDTA-PBS, and the rinse was collected in the same centrifuge tube. The liquid in the centrifuge tube containing the cells was diluted with EDTA-PBS up to a certain volume, followed by centrifugation (300 × g, room temperature, 15 minutes) and collection of the cells as a precipitate. The collected cells were incubated in ACK hemolysis buffer (15 mL/tube) (Gibco, Thermo-Fisher Scientific K.K.) at room temperature for 5 minutes to remove contaminating erythrocytes. ACK hemolysis buffer has a composition of 8,290 mg/l NH₄Cl, 1,000 mg/l KHCO₃, and 37 mg/l EDTA.Na₂.2H₂O. The resulting liquid was then diluted with EDTA-PBS up to a certain volume and centrifuged (200 × g, room temperature, 10 minutes or 100 × g, room temperature, 15 minutes) to collect cells, where the centrifugation step was repeated twice.

### (2) Culturing in a serum containing medium

The mononuclear cells collected in (1) were suspended in 1 mL of growth medium (Stemline(R) II Hematopoietic Stem Cell Expansion Medium (Sigma-Aldrich, Cat No. S0192) supplemented with 50 ng/mL VEGF165, 20 ng/mL TPO, 100 ng/mL Flt-3 ligand, 100 units/mL penicillin, 100 µg/mL streptomycin, and 0.5% FBS), and a portion thereof was used to count the cells using trypan blue. The concentration of the cells in the cell growth medium was adjusted to 1 × 10⁶/mL, and 2 mL of the cell suspension was plated into each well of a 6-well culture plate. The cells were cultured under common culture conditions (37°C, 5% CO2) for 5 days. After 5 days of culturing, the cells were collected into a centrifuge tube and centrifuged at 250 × g (about 1000 rpm) for 7 to 10 minutes three times to wash the cells. After centrifugation, the cells were counted to determine their survival rate. A sample was removed for a QC (validation) test, and the remaining cells were suspended in PlasmaLyte A and 2.5% human albumin serum and adjusted to a concentration of 8 × 10⁵ cells/mL. The resulting cell population may be referred to as "RE-01" below.

### Example 2: Characteristic evaluation of RE-01 cell population

### (1) Flow cytometric analysis

In this Example, the cell population obtained in Example 1 by culturing peripheral blood from the healthy human in the medium containing three factors and serum (referred to as "RE-01") was analyzed by flow cytometry to determine characteristics of the cell population.

The cells were suspended in FACS buffer (composition: 2 mM EDTA-PBS supplemented with 2% FBS) (1.5 × 10⁶ cells/300 µL of FACS buffer), and 10 µL of FC blocking reagent (Miltenyi Biotec K.K.) was added to the suspension and incubated at 4°C for 30 minutes. The incubated cell population was aliquoted in tubes for staining (100 µL/tube × 3 tubes). 2 µL of each primary antibody was added to each aliquot and incubated at 4°C for 20 minutes. The cells were then washed with 1 mL of FACS buffer twice, and the stained cells were suspended in FACS buffer (5 × 10⁵ cells /200 to 300 µL of FACS buffer). Flow cytometric analysis was performed on a BD FACSAria(TM) III cell sorter (Becton, Dickinson and Company).

Specifically, the expression of cell surface markers in the resulting cell population were determined using antibodies against the cell surface markers. All of the antibodies against the cell surface markers were commercially available as listed below.
Anti-CD206 antibody: PE/Cy7-labeled anti-human CD206 (MMR) antibody (BioLegend, Inc.);
Anti-CD34 antibody: PE-labeled anti-human CD34 antibody (BioLegend, Inc.);
Anti-CD3 antibody: Alexa Fluor700-labeled anti-CD3 antibody (BioLegend, Inc.);
Anti-CXCR4 antibody: APC-labeled anti-human CD184 (CXCR4) antibody (Becton, Dickinson and Company); and
Anti-CCR2 antibody: PerCP/Cy5.5-labeled human CD192 (CCR2) antibody (BioLegend, Inc.).

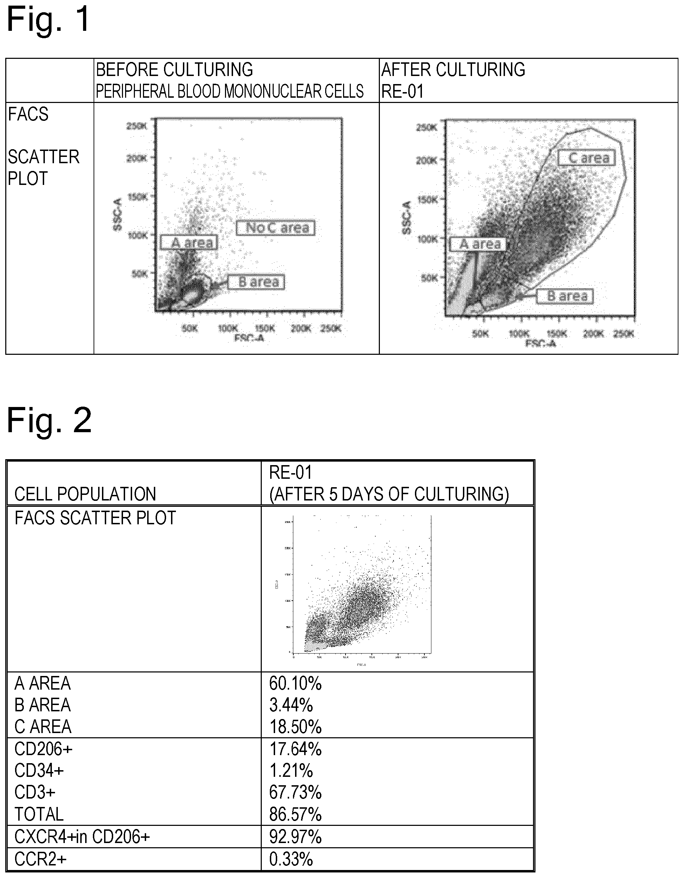

The results are shown in Fig. 1. As shown in Fig. 1, cells in RE-01 detected in the FACS scatter analysis were largely divided into three live cell populations (referred herein to as "A area", "B area", and "C area") on the basis of the size of cells (on the horizontal axis: FSC) and the density of cellular components, such as granules, present within the cells (on the vertical axis: SSC). Peripheral blood mononuclear cells before culturing were detected in A and B areas and were not detected in C area. A area includes mainly lymphocytes, and B area includes monocytes. In RE-01 obtained from the culturing in the growth medium (serum containing medium in Example 1 (2)), the detected area of endothelial progenitor cells (CD34 positive cells) moved from A area to B area, and cells including many M2 macrophages (CD206 positive cells) were newly detected in C area.

The result from flow cytometric analysis of RE-01 obtained by culturing mononuclear cells harvested from another healthy human in the medium containing three factors and serum as described in Example 1 is shown in Fig. 2. Cells in RE-01 derived from a healthy human different from the individual described above were also detected in A, B, and C areas. The total of CD206(+), CD34(+), or CD3(+) cells collectively represented 86.57% of the RE-01. CCR2(+) cells represented 0.33% of the RE-01; in other words, CCR2(-) cells represented 99.67%. Furthermore, CXCR4(+) cells in CD206(+) cells represented 92.97% of the RE-01.

### Example 3: Comparison of RE-01 cell population with MNC-QQ cell population

### (1) Culturing of RE-01 and MNC-QQ

Mononuclear cells were collected from a healthy human and a diabetic patient and cultured in the medium containing three factors and serum in a similar manner to Example 1 to obtain RE-01.

As a comparative example, mononuclear cells were isolated in a similar manner to that for obtaining RE-01 cells and then cultured in Stemline(R) II Hematopoietic Stem Cell Expansion Medium (Sigma-Aldrich, Cat No. S0192). This medium was prepared without serum by aseptically adding five factors of 50 ng/mL VEGF₁₆₅, 20 ng/mL TPO, 100 ng/mL Flt-3 ligand, 100 ng/mL SCF, and 20 ng/mL IL-6, as well as 100 units/mL penicillin and 100 µg/mL streptomycin. The concentration of the isolated cells was adjusted to 1 × 10⁶/mL in the growth medium, and 2 mL of the cell suspension was plated into each well of a 6-well culture plate. The cells were cultured under common culture conditions (37°C, 5% CO₂) for 7 days. After 7 days of culture, the cells were collected into a centrifuge tube and centrifuged at 250 × g (about 1000 rpm) for 7 to 10 minutes three times to wash the cells. After centrifugation, the cells were counted to determine their survival rate. A sample was removed for a QC (validation) test, and the remaining cells were suspended in PlasmaLyte A and 2.5% human albumin serum and adjusted to a concentration of 8 × 10⁵ cells/mL. The resulting cell population may hereinafter be referred to as "MNC-QQ".

### (2) Flow cytometric analysis

The cell population obtained in Example 1 by culturing peripheral blood from the diabetic patient in the medium containing three factors and serum and the cell population (MNC-QQ) obtained from the culturing in the serum-free medium containing five factors as described in (1) above were analyzed by flow cytometry to compare them. Specifically, the expression of cell surface markers in the resulting cell population were determined using antibodies against the cell surface markers.

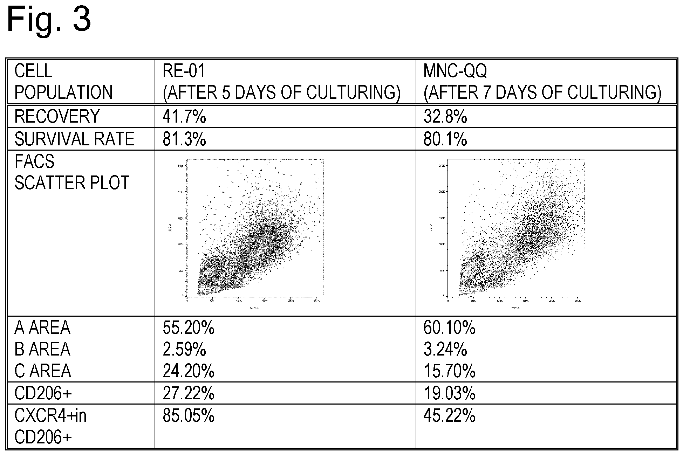

The results are shown in Fig. 3. RE-01 had a higher recovery and a higher survival rate after culturing than MNC-QQ. RE-01 further had a high proportion of cells detected in C area in which a cell population newly produced after culturing, and especially a high proportion of CD206+ cells which are a cell type responsible for the efficacy of the cell population. More surprisingly, RE-01 had a markedly high proportion of CXCR4+ cells in CD206+ cells, and the cell composition in RE-01 cell population was found to be different from that in MNC-QQ.

### (3) Ability to incorporate acLDL

The present inventors investigated an ability of the cell population (RE-01) obtained in Example 1 by culturing peripheral blood mononuclear cells from the diabetic patient in the medium containing three factors and serum and, as a comparative example, the cell population (MNC-QQ) obtained from the culturing in the serum-free medium containing five factors to incorporate acetylated low-density lipoprotein (acLDL).

RE-01 or MNC-QQ cells (1 × 10⁵ cells) collected in a 1.5 mL tube were suspended in 500 µL of a minimal essential medium (Stemline II), and 5 µL of acLDL labeled with Alexa Fluor488 was added to the tube. The suspension was then incubated at 37°C for 60 minutes. One mL of FACS buffer was added to the tube after the incubation and mixed through inversion. The tube was then centrifuged (250 × g, 4°C) for 5 minutes to collect cells as a precipitate. The collected cells (RE-01) were suspended in 300 µL of FACS buffer and stored under cooling in ice until measurement. DAPI (2 µL) was added to the tube immediately before measurement, and a proportion of cells that incorporated acLDL and DAPI was determined by the measurement on a flow cytometer.

The results are shown in Table 1 below.

**[Table 1]**

| Cell population | RE-01 (after 5 days of culturing) | MNC-QQ (after 7 days of culturing) |
|---|---|---|
| aLDL(+)/DAPI- | | |
| Total | 20.14% | 1.57% |
| in A area | 0.37% | 0.04% |
| in B area | 20.65% | 20.54% |
| in C area | 65.19% | 3.53% |

As shown in Table 1, it was revealed that total cells, particularly cells in C area incorporated more acLDL. This supports that in the two cell populations (RE-01 and MNC-QQ), the difference in cell composition as described in Example 2 leads to the difference in their function.

### (4) Ability to form blood vessels

In this Example, a cell population (RE-01) obtained by culturing mononuclear cells harvested from blood from a healthy human in the serum containing medium in a similar manner to Example 1 was used to investigate its ability to form blood vessels. The cell population (MNC-QQ) obtained from the culturing in the serum-free medium containing five factors was used as a comparative example. Cells from each population were co-cultured with human umbilical vein endothelial cells (HUVECs) (obtained from Lonza, Basel, Switzerland) which were used at the passage 8 to 10 and have the ability to form lumens. Basement membrane matrix (Corning(R) Matrigel(R) (Corning Incorporated, NY, United States)) was dispensed to a 96-well plate in 50 µl/well, and the plate was left at 37°C for 30 minutes to be coated. Each cell population was suspended in 500 µl of IMDM + 5 µl of DiI-Ac-LDL to label it with DiI-Ac-LDL and incubated at 37°C for 1 hour.

After labeling, each cell population was resuspended in PBS to 1 × 10³/20 µl, and HUVECs were resuspended in PBS to 5 × 10³/20 µl. These suspensions in which cell counts were adjusted were mixed together at a ratio of 1:1 of test cell population to HUVECs, and 50 µl of the mixture was added on Matrigel. The cells were photographed in a box in which a time-lapse fluorescent microscope (Olympus Corporation, Tokyo, Japan) was placed, while culturing at 37°C in 5% CO2 for 10 hours or more. After culturing, the numbers of formed lumens and fluorescently labeled test cells incorporated in the lumen structure were counted at a 100-fold magnification by visual observation.

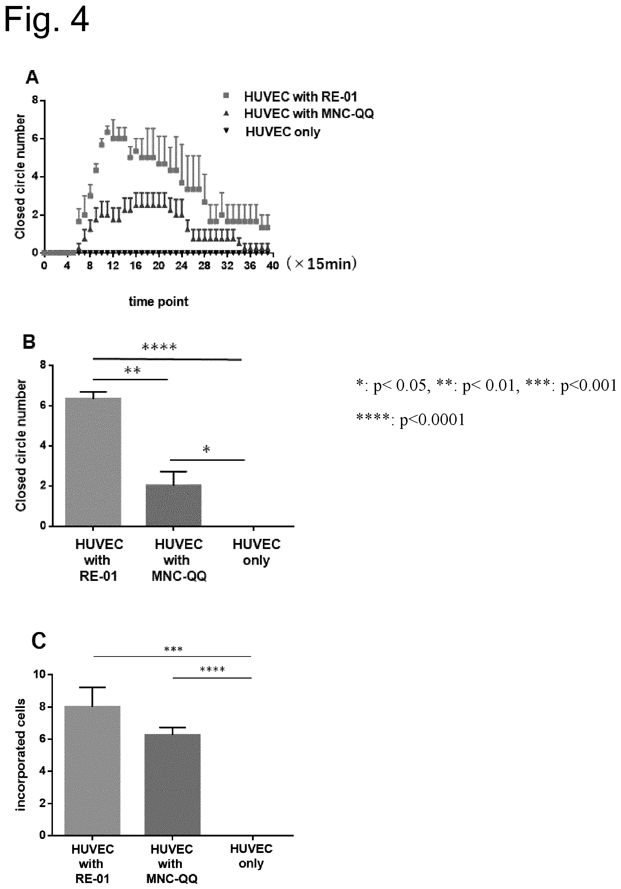

The results are shown in Fig. 4. Fig. 4A shows the change of the number of formed lumens over time from the start of culturing. Fig. 4B shows the number of formed lumens after 2.75 hours from the start of culturing. Fig. 4C shows the number of fluorescently labeled test cells incorporated in the lumen structure after 2.75 hours from the start of culturing. The ability of HUVECs co-cultured with RE-01 for a certain period of time to form lumens was higher than that of HUVECs cultured alone or HUVECs co-cultured with MNC-QQ. In addition, more cells in RE-01 were incorporated in the formed lumen structures. In other words, it was revealed that RE-01 in vitro had a higher in vitro ability to form blood vessels than MNC-QQ cultured in a serum-free medium for a longer period of time (for 7 hours). This supports that in the two cell populations (RE-01 and MNC-QQ), the difference in cell composition as described in Example 3 leads to the difference in their function.

### (5) Effect in a mouse model of lower limb ischemia

The cell population obtained by culturing mononuclear cells harvested from blood from a healthy human in a similar manner to Example 1 was used to investigate its therapeutic efficacy on lower limb ischemia in a mouse model of lower limb ischemia. The cell population (MNC-QQ) obtained from the culturing in the serum-free medium containing five factors was used as a comparative example.

### (6) Stability test

The cell population obtained by culturing mononuclear cells harvested from blood from a healthy human in a similar manner to Example 1 was used to investigate its stability from the time of collection to 72 hours on the basis of the cell count and survival rate. The cell population (MNC-QQ) obtained from the culturing in the serum-free medium containing five factors was used as a comparative example.

### Example 4: Culturing and characteristic evaluation of RE-01 cell population

In this Example, mononuclear cells were isolated from peripheral blood from a patient with severe lower limb ischemia and cultured. The resulting cell population was further subjected to characteristic evaluation.

### (1) Isolation of mononuclear cells

Mononuclear cells were isolated from peripheral blood from a patient with severe lower limb ischemia. The isolation was performed in a similar manner to Example 1.

Specifically, 100 mL of peripheral blood was harvested from a patient with severe lower limb ischemia into a BD Vacutainer(R) CPT(TM) (Becton, Dickinson and Company) blood collection tube for separating mononuclear cells. After blood collection, the blood collection tube was centrifuged as such, transported, received, and then stored under refrigeration until the start of culturing. The upper layer, which contained mononuclear cells and blood plasma, over the gel barrier in the CPT(TM) blood collection tube was collected into a centrifuge tube. The inside of the blood collection tube over the gel barrier was rinsed with a small volume of EDTA-PBS, and the rinse was collected in the same centrifuge tube. The liquid in the centrifuge tube containing the cells was diluted with EDTA-PBS up to a certain volume, followed by centrifugation (300 × g, room temperature, 15 minutes) and collection of the cells as a precipitate. The collected cells were incubated in ACK hemolysis buffer (15 mL/tube) (Gibco, Thermo-Fisher Scientific K.K.) at room temperature for 5 minutes to remove contaminating erythrocytes. ACK hemolysis buffer has a composition of 8,290 mg/l NH₄Cl, 1,000 mg/l KHCO₃, and 37 mg/l EDTA.Na₂.2H₂O. The resulting liquid was then diluted with EDTA-PBS up to a certain volume and centrifuged (200 × g, room temperature, 10 minutes or 100 × g, room temperature, 15 minutes) to collect cells, where the centrifugation step was repeated twice.

### (2) Culturing in a serum containing medium

The isolated mononuclear cells were cultured in a serum containing medium in a similar manner to Example 1, except that Iscove's Modified Dulbecco's Medium (IMDM) was used as a basal medium instead of Stemline(R) and that human albumin serum was also added along with FBS as a serum.

Specifically, the mononuclear cells collected in (1) were suspended in 1 mL of growth medium (Iscove's Modified Dulbecco's Medium (IMDM) supplemented with 50 ng/mL VEGF₁₆₅, 20 ng/mL TPO, 100 ng/mL Flt-3 ligand, 100 units/mL penicillin, 100 µg/mL streptomycin, 0.5% FBS, and 0.5% human albumin serum), and a portion thereof was used to count the cells using trypan blue. The concentration of the cells in the cell growth medium was adjusted to 1 × 10⁶/mL, and 2 mL of the cell suspension was plated into each well of a 6-well culture plate. The cells were cultured under common culture conditions (37°C, 5% CO₂) for 5 days. After 5 days of culturing, the cells were collected into a centrifuge tube and centrifuged at 250 × g (about 1000 rpm) for 7 to 10 minutes three times to wash the cells. After centrifugation, the cells were counted to determine their survival rate. A sample was removed for a QC (validation) test, and the remaining cells were suspended in PlasmaLyte A and 2.5% human albumin serum and adjusted to a concentration of 8 × 10⁵ cells/mL.

### (3) Flow cytometric analysis

In this Example, the cell population obtained in (1) and (2) by culturing mononuclear cells isolated from peripheral blood from the patient with severe lower limb ischemia in the medium containing three factors and serum (referred to as "RE-01" as in Example 1) was analyzed by flow cytometry to determine characteristics of the cell population.

The cells were suspended in FACS buffer (composition: 2 mM EDTA-PBS supplemented with 2% FBS) (1.5 × 10⁶ cells/300 µL of FACS buffer), and 10 µL of FC blocking reagent (Miltenyi Biotec K.K.) was added to the suspension and incubated at 4°C for 30 minutes. The incubated cell population was aliquoted in tubes for staining (100 µL/tube × 3 tubes). Two µL of each primary antibody was added to each aliquot and incubated at 4°C for 20 minutes. The cells were then washed with 1 mL of FACS buffer twice, and the stained cells were suspended in FACS buffer (5 × 10⁵ cells /200 to 300 µL of FACS buffer). Flow cytometric measurement was performed on a BD FACSAria(TM) III cell sorter (Becton, Dickinson and Company).

Specifically, the expression of cell surface markers in the resulting cell population were determined using antibodies against the cell surface markers. All of the antibodies against the cell surface markers were commercially available as listed below.
Anti-CD206 antibody: PE/Cy7-labeled anti-human CD206 (MMR) antibody (BioLegend, Inc.);
Anti-CD34 antibody: PE-labeled anti-human CD34 antibody (BioLegend, Inc.);
Anti-CD3 antibody: Alexa Fluor700-labeled anti-CD3 antibody (BioLegend, Inc.);
Anti-CXCR4 antibody: APC-labeled anti-human CD184 (CXCR4) antibody (Becton, Dickinson and Company); and
Anti-CCR2 antibody: PerCP/Cy5.5-labeled human CD192 (CCR2) antibody (BioLegend, Inc.).

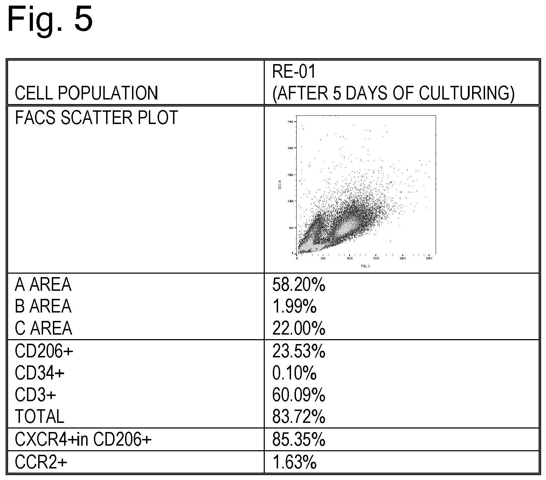

The results are shown in Fig. 5. As shown in Fig. 5, cells in the cell population obtained by culturing mononuclear cells isolated from peripheral blood from the patient with severe lower limb ischemia were also detected in A, B, and C areas as in Example 2. The total of CD206(+), CD34(+), or CD3(+) cells collectively represented 83.72% of the cell population, and CCR2(+) cells represented 1.63%. In other words, CCR2(-) cells represented 98.37% of the cell population. Furthermore, CXCR4(+) cells in CD206(+) cells represented 83.72% of the cell population.

The results reveal that RE-01 cell population, particularly RE-01 cell population in which the total of CD206(+), CD34(+), or CD3(+) cells collectively represents 80% or more, CCR2(-) cells represent 95% or more, and CXCR4(+) cells in CD206(+) cells represent 80% or more, can be also obtained by culturing peripheral blood mononuclear cells from a patient with severe lower limb ischemia in the medium containing three factors and serum, in a similar manner to the culturing of peripheral blood mononuclear cells from the healthy human or the diabetic patient in Example 1.

### INDUSTRIAL APPLICABILITY

The present invention provides a novel cell population having high abilities to regenerate blood vessels and to heal wounds. The cell population of the present invention can be used as a therapeutic agent for patients with an incurable disease, limb ischemia disease, for example. This achieves the improvement of the Quality of Life (QOL) of patients, such as avoidance of amputation, and innovation of life, such as decreased burden of nursing care, resulting in a significant contribution to society. The provision of novel cell population leads to the establishment of a new therapeutic strategy and can bring about findings regarding vascular lesions in general. Furthermore, this cell population surprisingly achieves high efficacy as well as a stably higher recovery and survival rate in a shorter culture period and is more stable after manufacturing than a cell population obtained by the existing QQ-MNC method, possibly giving great benefits industrially.

## Claims

1. A cell population obtained by culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

2. The cell population according to claim 1, wherein the medium contains three or four factors selected from stem cell factor, interleukin-6, FMS-like tyrosine kinase 3 ligand, thrombopoietin, and vascular endothelial growth factor.

3. The cell population according to claim 1 or 2, wherein the serum is bovine or human serum.

4. The cell population according to any one of claims 1 to 3, wherein the serum is contained in the medium at a concentration from 0.5 vol% to 10 vol%.

5. The cell population according to any one of claims 1 to 4, wherein the total of CD206(+), CD34(+), or CD3(+) cells collectively represents 60% or more of the cell population, and CCR2(-) cells represent 95% or more of the cell population.

6. The cell population according to claim 5, wherein CXCR4(+) cells in CD206(+) cells represent 50% or more of the cell population.

7. The cell population according to claim 5 or 6, wherein CXCR4(+) cells in CD206(+) cells represent 80% or more of the cell population.

8. A method of obtaining the cell population according to any one of claims 1 to 7, comprising culturing mononuclear cells derived from bone marrow, umbilical cord blood, or peripheral blood in a medium containing serum and four or less of factors selected from the group consisting of stem cell factor, interleukin-6, FMS-like tyrosine kinase 3 ligand, thrombopoietin, and vascular endothelial growth factor.

9. The method according to claim 8, wherein the total of CD206(+), CD34(+), or CD3(+) cells collectively represents 60% or more of the cell population, and CCR2(-) cells represent 95% or more of the cell population.

10. The method according to claim 8 or 9, wherein CXCR4(+) cells in CD206(+) cells represent 50% or more of the cell population.

11. The method according to any one of claims 8 to 10, wherein the medium contains three or four factors selected from stem cell factor, interleukin-6, FMS-like tyrosine kinase 3 ligand, thrombopoietin, and vascular endothelial growth factor.

12. The method according to any one of claims 8 to 11, wherein the serum is fetal bovine serum.

13. The method according to any one of claims 8 to 12, wherein the serum is contained in the medium at a concentration from 0.5 vol% to 10 vol%.

14. A therapeutic composition for ischemic diseases, inflammatory diseases, or refractory wounds, comprising the cell population according to any one of claims 1 to 7.

15. The therapeutic composition according to claim 14, wherein the ischemic disease is limb ischemia.

16. The therapeutic composition according to claim 14 or 15, wherein the ischemic disease is limb ischemia with ulcers.

17. The therapeutic composition according to any one of claims 14 to 16, promoting vascularization and/or wound healing.
